# EUROPÄISCHE PATENTSCHRIFT

(11) **EP 1 412 488 B1**
(45) Veröffentlichungstag und Bekanntmachung des Hinweises auf die Patenterteilung: **10.10.2007**
(21) Anmeldenummer: 02764826.0
(22) Anmeldetag: 31.07.2002
(51) Int. Cl.: C12N 9/02, C12N 15/53, C12N 5/10

(54) **VERFAHREN ZUR HERSTELLUNG VON ARACHIDONSÄURE IN TRANSGENEN ORGANISMEN**
METHOD FOR PRODUCING ARACHIDONIC ACID IN TRANSGENIC ORGANISMS
PROCEDE DE PRODUCTION D'ACIDE ARACHIDONIQUE DANS DES ORGANISMES TRANSGENIQUES

(30) Priorität: 31.07.2001 DE 10137374
(43) Veröffentlichungstag der Anmeldung: 28.04.2004
(73) Patentinhaber: Leibniz-Institut für Pflanzengenetik und Kulturpflanzenforschung (IPK), 06466 Gatersleben (DE)
(72) Erfinder: FEUSSNER, Ivo, 37085 Göttingen (DE); HORNUNG, Ellen, 37077 Göttingen (DE); PERNSTICH, Christian, 13509 Berlin (DE); KORFEI, Martina, 35094 Lahntal-Sarnau (DE); KINDL, Helmut, 35043 Marburg (DE)
(74) Vertreter: Neuefeind, Regina
(86) Internationale Anmeldenummer: PCT/EP2002/008555
(87) Internationale Veröffentlichungsnummer: WO 2003/012092

(56) Entgegenhaltungen:
- WO-A-02/26946
- KNUTZON D S ET AL: "Identification of Delta5 - desaturase from Mortierella alpina by heterologous expression in Bakers yeast and canola" JOURNAL OF BIOLOGICAL CHEMISTRY, AMERICAN SOCIETY OF BIOLOGICAL CHEMISTS, BALTIMORE, MD, US, Bd. 273, Nr. 45, 6. November 1998 (1998-11-06), Seiten 29360-29366, XP002106760
- LEONARD AMANDA E ET AL: "cDNA cloning and characterization of human DELTA5-desaturase involved in the biosynthesis of arachidonic acid." BIOCHEMICAL JOURNAL, Bd. 347, Nr. 3, 1. Mai 2000 (2000-05-01), Seiten 719-724, XP002223219

### SEQUENZPROTOKOLL

<110> Institut für Pflanzengenetik und Kulturpflanzenfor
<120> Verfahren zur Herstellung von Arachidonsäure in transgenen Organismen
<130> I 7376
<140>
   <141>
<150> DE 101 37 374.0
   <151> 2001-07-31
<160> 6
<170> Patent In Ver. 2.1
<210> 1
   <211> 1514
   <212> DNA
   <213> Phytophthera megasperma
<220>
   <221> CDS
   <222> (1)..(1431)
<400> 1
   atg gcc ccc atc gag act gtc aaa gac gcc aac gag ggc ctg cac cag      48
   Met Ala Pro Ile Glu Thr Val Lys Asp Ala Asn Glu Gly Leu His Gln
     1               5                  10                  15
   cgc aag ggc gcc gct gcc gcc tcc aag gac acg acc acc ttc acg tgg      96
   Arg Lys Gly Ala Ala Ala Ala Ser Lys Asp Thr Thr Thr Phe Thr Trp
                20                  25                  30
   cag gac gtg gcc aag cac aac acg gcc aag agc gcc tgg gtc acc atc     144
   Gln Asp Val Ala Lys His Asn Thr Ala Lys Ser Ala Trp Val Thr Ile
            35                  40                  45
   cgc ggc gtc gtc tac gac gtg acc gaa tgg gcc gac cgc cac ccc gga     192
   Arg Gly Val Val Tyr Asp Val Thr Glu Trp Ala Asp Arg His Pro Gly
        50                  55                  60
   ggc cgc gag ctc gtg ctg ctg cac tcg ggc cgt gag tgc acg gac acg     240
   Gly Arg Glu Leu Val Leu Leu His Ser Gly Arg Glu Cys Thr Asp Thr
    65                  70                  75                  80
   ttc gac tcg tac cac ccg ttc tcc gac cgc gcg gac aag atc ctg gcc     288
   Phe Asp Ser Tyr His Pro Phe Ser Asp Arg Ala Asp Lys Ile Leu Ala
                    85                  90                  95
   aag tac gcc atc ggc aag ctc gtg ggc ggc tcc gag ttc ccc acg tac     336
   Lys Tyr Ala Ile Gly Lys Leu Val Gly Gly Ser Glu Phe Pro Thr Tyr
               100                 105                 110
   aag ccc gac acg ggc ttc tac aag gag tgc tgc gac cgc gtc aac cag     384
   Lys Pro Asp Thr Gly Phe Tyr Lys Glu Cys Cys Asp Arg Val Asn Gln
           115                 120                 125
   tac ttc aag gac aac aag ctg gac ccg cgc agc ccc tac tcg ggt ctg     432
   Tyr Phe Lys Asp Asn Lys Leu Asp Pro Arg Ser Pro Tyr Ser Gly Leu
       130                 135                 140
   tgg cgc atg atc ctc gtg gcc atc gtc ggc gct gtg gcc tac atg ggc      480
   Trp Arg Met Ile Leu Val Ala Ile Val Gly Ala Val Ala Tyr Met Gly
   145                 150                 155                 160
   atg aac cag ctg ctg ccg ggc aac atc tac gcg cac tac gcg tgg ggc      528
   Met Asn Gln Leu Leu Pro Gly Asn Ile Tyr Ala His Tyr Ala Trp Gly
                   165                 170                 175
   gcg ctc ttc ggc gtg tgc caa gcg ctg ccg ctg ctg cac gtc atg cac      576
   Ala Leu Phe Gly Val Cys Gln Ala Leu Pro Leu Leu His Val Met His
               180                 185                 190
   gac gcg tcg cac gct gcc atc acc agc agc ccc acg ggc tgg cgg ctc      624
   Asp Ala Ser His Ala Ala Ile Thr Ser Ser Pro Thr Gly Trp Arg Leu
           195                 200                 205
   atc ggc cgc ctc gcg atg gac tgg gtg gcc ggc gcc aac atg gtc tcg      672
   Ile Gly Arg Leu Ala Met Asp Trp Val Ala Gly Ala Asn Met Val Ser
       210                 215                 220
   tgg ctc aac cag cac gtg gtg ggc cac cac atc tac acg aac gtg gca      720
   Trp Leu Asn Gln His Val Val Gly His His Ile Tyr Thr Asn Val Ala
   225                 230                 235                 240
   ggc gcg gac ccg gat ctg ccc gtg gac ttc aag agc gac gtg cgc cgc      768
   Gly Ala Asp Pro Asp Leu Pro Val Asp Phe Lys Ser Asp Val Arg Arg
                   245                 250                 255
   atc gtg tac cgc cag gtg ctg ctg ccc att tac aag tac cag cac ctg      816
   Ile Val Tyr Arg Gln Val Leu Leu Pro Ile Tyr Lys Tyr Gln His Leu
               260                 265                 270
   tac ctg ccg ccg ctc tac ggc gtg ctg ggc ctc aag ttc cgc gtg cag      864
   Tyr Leu Pro Pro Leu Tyr Gly Val Leu Gly Leu Lys Phe Arg Val Gln
           275                 280                 285
   gac gtc ttc gag acg ttc gtc acg ctg acg aat ggc ccg ctg cgc gtg      912
   Asp Val Phe Glu Thr Phe Val Thr Leu Thr Asn Gly Pro Leu Arg Val
       290                 295                 300
   aac ccg ctc tcg gtg ggc gac tgg gcc gag atg atc ctg tcc aag gcc      960
   Asn Pro Leu Ser Val Gly Asp Trp Ala Glu Met Ile Leu Ser Lys Ala
   305                 310                 315                 320
   ttc tgg gtc ttc tac cgc atc tac ctc ccg ctg gct gtg ctc cag gtg     1008
   Phe Trp Val Phe Tyr Arg Ile Tyr Leu Pro Leu Ala Val Leu Gln Val
                   325                 330                 335
   gac ccc gct cgc ttc tgg ggc gtc ttc ttc ctc gcc gag ttc tcc acg     1056
   Asp Pro Ala Arg Phe Trp Gly Val Phe Phe Leu Ala Glu Phe Ser Thr
               340                 345                 350
   ggc tgg tac ctg gcc ttc aac ttc cag gtg agc cac gtg tcc acg gcc     1104
   Gly Trp Tyr Leu Ala Phe Asn Phe Gln Val Ser His Val Ser Thr Ala
           355                 360                 365
   tgc gag tac ccg ggc ggt gac gag gaa gtg acg tcc atc gac gac gag     1152
   Cys Glu Tyr Pro Gly Gly Asp Glu Glu Val Thr Ser Ile Asp Asp Glu
       370                 375                 380
   tgg gcc atc tcg cag gtc aag tcg tcg gtg gac tac ggc cac ggc tcg     1200
   Trp Ala Ile Ser Gln Val Lys Ser Ser Val Asp Tyr Gly His Gly Ser
   385                 390                 395                 400
   ttc atc acg acg ttc ctc acg ggc gcg ctc aac tac cag gtg acg cac    1248
   Phe Ile Thr Thr Phe Leu Thr Gly Ala Leu Asn Tyr Gln Val Thr His
                   405                 410                 415
   cac ctc ttc ccg ggc gtc tcg cag tac cat tac ccg gcc att gcg ccg    1296
   His Leu Phe Pro Gly Val Ser Gln Tyr His Tyr Pro Ala Ile Ala Pro
               420                 425                 430
   ctc atc ctc gac gtg tgc cac aag tac aag gtc aag tac aac gtg ctc    1344
   Leu Ile Leu Asp Val Cys His Lys Tyr Lys Val Lys Tyr Asn Val Leu
           435                 440                 445
   ccg gac ttc acg gcg gcc atg gcc ggc cac ttc gac cac ctt gtc att    1392
   Pro Asp Phe Thr Ala Ala Met Ala Gly His Phe Asp His Leu Val Ile
       450                 455                 460
   atg ggc aag atg ggc aag cgc gtg acc atc cac atg ggc taagccagca     1441
   Met Gly Lys Met Gly Lys Arg Val Thr Ile His Met Gly
   465                 470                 475
   gcggtcggtt gaggacaagg ccgtcttgca gctggactgc gactgctggt aagctttggc 1501
   tggataacgc ggg                                                     1514
<210> 2
   <211> 477
   <212> PRT
   <213> Phytophthera megasperma
<400> 2
   Met Ala Pro Ile Glu Thr Val Lys Asp Ala Asn Glu Gly Leu His Gln
    1               5                  10                  15
   Arg Lys Gly Ala Ala Ala Ala Ser Lys Asp Thr Thr Thr Phe Thr Trp
               20                  25                  30
   Gln Asp Val Ala Lys His Asn Thr Ala Lys Ser Ala Trp Val Thr Ile
           35                  40                  45
   Arg Gly Val Val Tyr Asp Val Thr Glu Trp Ala Asp Arg His Pro Gly
       50                  55                  60
   Gly Arg Glu Leu Val Leu Leu His Ser Gly Arg Glu Cys Thr Asp Thr
   65                  70                  75                  80
   Phe Asp Ser Tyr His Pro Phe Ser Asp Arg Ala Asp Lys Ile Leu Ala
                   85                  90                  95
   Lys Tyr Ala Ile Gly Lys Leu Val Gly Gly Ser Glu Phe Pro Thr Tyr
               100                 105                 110
   Lys Pro Asp Thr Gly Phe Tyr Lys Glu Cys Cys Asp Arg Val Asn Gln
           115                 120                 125
   Tyr Phe Lys Asp Asn Lys Leu Asp Pro Arg Ser Pro Tyr Ser Gly Leu
       130                 135                 140
   Trp Arg Met Ile Leu Val Ala Ile Val Gly Ala Val Ala Tyr Met Gly
   145                 150                 155                 160
   Met Asn Gln Leu Leu Pro Gly Asn Ile Tyr Ala His Tyr Ala Trp Gly
   165 170 175
   Ala Leu Phe Gly Val Cys Gln Ala Leu Pro Leu Leu His Val Met His
   180 185 190
   Asp Ala Ser His Ala Ala Ile Thr Ser Ser Pro Thr Gly Trp Arg Leu
   195 200 205
   Ile Gly Arg Leu Ala Met Asp Trp Val Ala Gly Ala Asn Met Val Ser
   210 215 220
   Trp Leu Asn Gln His Val Val Gly His His Ile Tyr Thr Asn Val Ala
   225 230 235 240
   Gly Ala Asp Pro Asp Leu Pro Val Asp Phe Lys Ser Asp Val Arg Arg
   245 250 255
   Ile Val Tyr Arg Gln Val Leu Leu Pro Ile Tyr Lys Tyr Gln His Leu
   260 265 270
   Tyr Leu Pro Pro Leu Tyr Gly Val Leu Gly Leu Lys Phe Arg Val Gln
   275 280 285
   Asp Val Phe Glu Thr Phe Val Thr Leu Thr Asn Gly Pro Leu Arg Val
   290 295 300
   Asn Pro Leu Ser Val Gly Asp Trp Ala Glu Met Ile Leu Ser Lys Ala
   305 310 315 320
   Phe Trp Val Phe Tyr Arg Ile Tyr Leu Pro Leu Ala Val Leu Gln Val
   325 330 335
   Asp Pro Ala Arg Phe Trp Gly Val Phe Phe Leu Ala Glu Phe Ser Thr
   340 345 350
   Gly Trp Tyr Leu Ala Phe Asn Phe Gln Val Ser His Val Ser Thr Ala
   355 360 365
   Cys Glu Tyr Pro Gly Gly Asp Glu Glu Val Thr Ser Ile Asp Asp Glu
   370 375 380
   Trp Ala Ile Ser Gln Val Lys Ser Ser Val Asp Tyr Gly His Gly Ser
   385 390 395 400
   Phe Ile Thr Thr Phe Leu Thr Gly Ala Leu Asn Tyr Gln Val Thr His
   405 410 415
   His Leu Phe Pro Gly Val Ser Gln Tyr His Tyr Pro Ala Ile Ala Pro
   420 425 430
   Leu Ile Leu Asp Val Cys His Lys Tyr Lys Val Lys Tyr Asn Val Leu
   435 440 445
   Pro Asp Phe Thr Ala Ala Met Ala Gly His Phe Asp His Leu Val Ile
   450 455 460
   Met Gly Lys Met Gly Lys Arg Val Thr Ile His Met Gly
   465 470 475
<210> 3
   <211> 27
   <212> DNA
   <213> Künstliche Sequenz
<220>
   <223> Beschreibung der künstlichen Sequenz: Oligonukleotidsonde bzw. Oligonukleotidprimer
<400> 3
   gtggccaagc acaacacggc caagagc 27
<210> 4
   <211> 30
   <212> DNA
   <213> Künstliche Sequenz.
<220>
   <223> Beschreibung der künstlichen Sequenz: Oligonukleotidsonde bzw. Oligonukleotidprimer
<400> 4
   accatccgcg gcgtcgtcta cgacgtgacc 30
<210> 5
   <211> 31
   <212> DNA
   <213> Künstliche Sequenz
<220>
   <223> Beschreibung der künstlichen Sequenz: Oligonukleotidsonde bzw. Oligonukleotidprimer
<400> 5
   ggatccatgg cccccatcga gactgtcaaa g 31
<210> 6
   <211> 32
   <212> DNA
   <213> Künstliche Sequenz
<220>
   <223> Beschreibung der künstlichen Sequenz: Oligonukleotidsonde bzw. Oligonukleotidprimer
<400> 6
   gcatgccccg cgttatccag ccaaagctta cc 32

## Beschreibung

Die Erfindung betrifft ein Verfahren zur Herstellung von Arachidonsäure in transgenen Organismen, insbesondere in transgenen Pflanzen und Hefen. Des weiteren betrifft die Erfindung DNA-Sequenzen, die für ein Protein mit der enzymatischen Aktivität einer Δ5-Desaturase aus *Phytophthora megasperma* kodieren. Ferner betrifft die Erfindung transgene Pflanzen und Pflanzenzellen sowie transgene Hefen, die ein eine erfindungsgemäße DNA-Sequenz umfassendes Nukleinsäuremolekül enthalten und auf Grund dessen im Vergleich zu Wildtypzellen eine gesteigerte Arachidonsäuresynthese aufweisen, sowie Ernteprodukte und Vermehrungsmaterial der transgenen Pflanzen.

Ungesättigte Fettsäuren sind essentielle Komponenten, die für eine normale zelluläre Funktion benötigt werden. Dabei erfüllen ungesättigte Fettsäuren vielfältige Funktionen, z.B. bei der Membranfluidität bis hin zu ihrer Wirkung als Signalmoleküle. Insbesondere die Klasse von Fettsäuren, die als polyungesättigte Fettsäuren (polyunsaturated fatty acids, PUFA) bezeichnet werden, haben in letzter Zeit großes Interesse als pharmazeutische und nahrungsmitteltechnische Verbindungen hervorgerufen. PUFA können als Fettsäuren mit einer Länge von 18 oder mehr Kohlenstoffatomen definiert werden, wobei die PUFA zwei oder mehr Doppelbindungen enthalten. Diese Doppelbindungen werden durch Fettsäurespezifische Desaturase-Enzyme eingefügt.

Die PUFA können in zwei Gruppen klassifiziert werden, n-6 oder n-3, abhängig von der Position (n) der dem Methyl-Ende der Fettsäure nächsten Doppelbindung. So wird -γ-Linolensäure (18:3Δ^{6,9,12}) als 18:3, n-6 PUFA klassifiziert, während α-Linolensäure (18:3Δ^{9,12,15}) eine 18:3, n-3 PUFA ist. Viele PUFA sind ebenfalls essentielle Fettsäuren, die in der Nahrung enthalten sein müssen, um eine normale Entwicklung in Säugern, die die primäre essentielle PUFA-Fettsäure Linolsäure (18:2, n-6) nicht synthetisieren können, zu gewährleisten. Seit kurzem richtet sich das Interesse auch auf die C20-Fettsäure Arachidonsäure (20:4, n-6), für die gezeigt wurde, dass sie für die neonatale Gesundheit, einschließlich der Entwicklung des Gehirns und der Augen, von Bedeutung ist.

Arachidonsäure (5,8,11,14-Eicosatetraensäure) wird zusammen mit Linolsäure und Linolensäure zu den essentiellen Fettsäuren gerechnet. Die enzymatische Oxidation der Arachidonsäure führt zu einer Vielzahl biochemisch bedeutender Verbindungen wie den Prostaglandinen, Thromboxanen, Prostacyclinen, Leukotrienen und Lipoxinen, die unter der Bezeichnung Eicosanoide zusammengefaßt werden.

C20-Fettsäuren wie 20:4, n-6 werden durch nacheinanderfolgende Desaturation an Δ6, Elongation nach C-20, und weitere Desaturation an Δ5 aus 18:2, n-6, in der Nahrung synthetisiert.

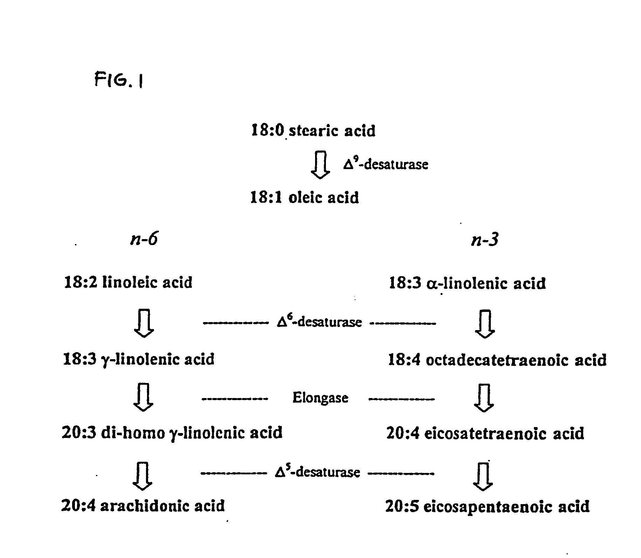

Abbildung 1 zeigt eine schematisierte PUFA-Biosynthese in Tieren. Primäre n-6 und n-3 Fettsäuren (in der Form von Linolsäure und α-Linolensäure) müssen in der Nahrung bereitgestellt werden, da Tiere nicht in der Lage sind, Ölsäure weiter zu desaturieren. Die in diesem Syntheseweg erforderlichen Enzymaktivitäten sind gezeigt.

Während Arachidonsäure das Hauptstoffwechselprodukt des n-6-Biosyntheseweges in Säugern ist, sind die Hauptendprodukte des n-3 Weges Eicosapentaensäure (20:5, n-3) und Docosahexansäure (22:6, n-3).

Arachidonsäure findet man in größeren Mengen u.a. in der Leber sowie in der Nebenniere. Sie wird auch in dem filamentösen Pilz *Mortierella alpina* und der Rotalge *Porphyridium cruentrum* synthetisiert. Eicosapentaensäure findet man in Fischöl und anderen marinen Organismen. Jedoch sind viele dieser Quellen für die Verwendung durch den Menschen nicht auf einfache Weise erhältlich. Da Pflanzenöle gegenwärtig die größte Quelle für PUFA in der menschlichen Nahrung sind, könnte die Modifikation von Fettsäure-Biosynthesewegen durch genetische Manipulation zwecks Erzeugung der gewünschten PUFA in einer Ölsaatpflanze eine wirtschaftliche Quelle für diese wichtigen Fettsäuren liefern. Während der letzten Jahre konnten Fettsäure-Desaturasegene aus verschiedenen Organismen kloniert werden (Napier et al. (1999) Curr. Opin. Plant Biol. 2:123-127).

Allerdings sind die im Stand der Technik beschriebenen Δ5-Desaturase-Enzyme nicht spezifisch für Dihomo-γ-Linolensäure (dihomo-γ-linolenic acid, DGLA; 20:3, n-6). So zeigen bisherige Arbeiten über entsprechende Gene bzw. Genprodukte, dass die im Stand der Technik bekannten Δ5-Desaturase-Enzyme nicht nur 20:3-, sondern auch 20:2-, 20:1- und in manchen Fällen sogar C18-Fettsäuren umsetzen (siehe beispielsweise Beaudoin et al. (2000) Proc. Natl. Acad. Sci. USA 97: 6421-6426; Cho et al. (1999) J. Biol. Chem. 274: 37335-37339; Knutzon et al. (1998) J. Biol. Chem. 273: 29360-29366; Saito et al. (2000) Eur. J. Biochem. 267: 1813-1818). Hieraus ergeben sich wichtige Nachteile, die man möglichst vermeiden möchte. So entstehen angesichts der fehlenden Spezifität der Enzyme des Standes der Technik immer Fettsäuregemische, die man gerne vermeiden möchte. So werden nicht nur definierte langkettige PUFA gebildet, sondern auch nicht literaturbekannte oder wertlose Nebenprodukte wie Picolinsäure (siehe Knutzon et al., Saito et al., vide supra). Derartige Enzyme sind somit beispielsweise für den Einsatz in der Lebensmittelindustrie nicht geeignet.

Gewünscht ist ein Enzym, welches die gezielte Herstellung und Akkumulation einer oder weniger Fettsäuren in einem transgenen Organismus ermöglicht. Durch Bereitstellung eines Enzyms mit Substratspezifität für 20:3-Fettsäuren, insbesondere Dihomo-γ-Linolensäure, ließen sich die Nachteile des Standes der Technik überwinden und gezielt 20:4-Fettsäuren, insbesondere Arachidonsäure, in transgenen Organismen herstellen.

Es ist jetzt überraschend gelungen, ein Gen für ein bisher unbekanntes Protein mit der enzymatischen Aktivität einer Δ5-Desaturase aus dem Pilz *Phytophthora megasperma* zu isolieren. Dieses Enzym ist im Unterschied zu den im Stand der Technik beschriebenen Enzymen mit Δ5-Desaturase-Aktivität für Dihomo-γ-Linolensäure spezifisch. Aus diesem Grund läßt sich das hier beschriebene Gen besonders vorteilhaft für die Expression der Δ5-Desaturase und damit für die enzymatische Herstellung von Arachidonsäure in transgenen Pflanzen und Hefezellen einsetzen.

Die erfindungsgemäßen Enzyme unterscheiden sich von den im Stand der Technik beschriebenen Δ5-Desaturasen eindeutig hinsichtlich der Substratspezifität. Während die bekannten Enzyme neben 20:3 auch andere Fettsäuren umsetzen, setzen die Enzyme der Erfindung nur 20:3 um. Diese hohe Substratspezifität war angesichts der aus dem Stand der Technik bekannten Spezifitäten von anderen Enzymen völlig unerwartet. Anhand einfacher Substratspezifitätsanalysen, z.B. bei Expression in Hefe, lassen sich die Enzyme der Erfindung somit von denen des Standes der Technik unterscheiden.

Die vorliegende Erfindung betrifft somit DNA-Sequenzen, die für ein Protein mit der enzymatischen Aktivität einer Δ5-Desaturase kodieren, wobei die Aktivität für Dihomo-γ-Linolensäure spezifisch ist.

Eine für Dihomo-γ-Linolensäure spezifische Aktivität bedeutet im Rahmen der vorliegenden Erfindung, dass die durch die erfindungsgemäßen DNA-Sequenzen kodierten Proteine mit der enzymatischen Aktivität einer Δ5-Desaturase im wesentlichen ausschließlich für Dihomo-γ-Linolensäure (20:3, DHLA) spezifisch sind und im wesentlichen keine anderen Fettsäuren umsetzen. Besonders bevorzugt bedeutet Spezifität für DHLA, dass die Δ5-Desaturase ausschließlich DHLA als Substrat umsetzt. Anders gesagt, bedeutet DHLA-Spezifität im Sinne der Erfindung auch, dass das Enzym weder 20:2-, noch 20:1- noch C18-Fettsäuren in nennenswerten Mengen, bevorzugt überhaupt nicht, umsetzt.

Im Unterschied hierzu setzen die im Stand der Technik beschriebenen Δ5-Desaturasen aus Mortierella alpina und Dictyostelium discoideum auch andere Fettsäuren um, was die oben beschriebenen Nachteile mit sich bringt.

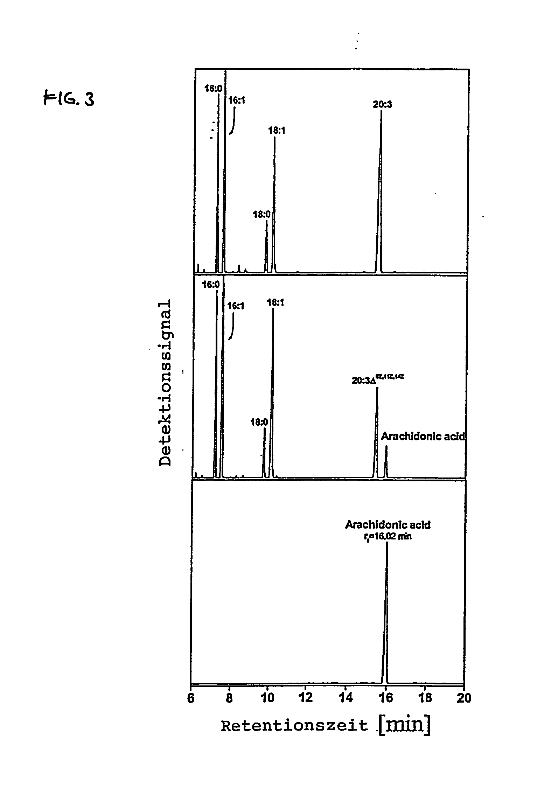

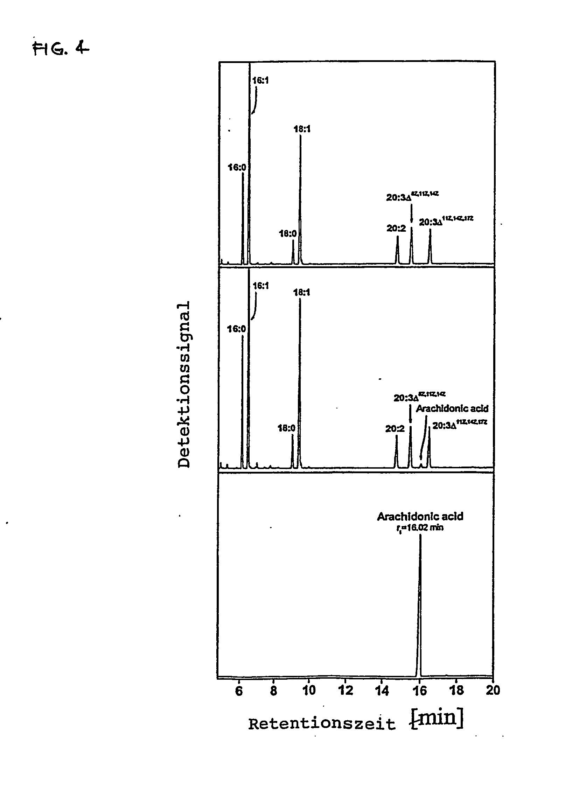

Für den Fachmann ist auf einfache Weise feststellbar, ob eine Δ5-Desaturase die erfindungsgemäße DHLA-Spezifität aufweist, es sich bei dem Enzym also um eine Δ5-Desaturase im Sinne der vorliegenden Erfindung handelt. So zeigt sich für den Fachmann beispielsweise in einfachen Standard-Fütterungsexperimenten, ob ein Enzym die hohe Substratspezifität für DHLA aufweist (siehe z.B. Abbildungen 3 und 4). Werden in einem Fütterungsexperiment z.B. verschiedene C20-Fettsäuren wie z.B. 20:2Δ^{11,14}, 20:3Δ^{8,11,14}(DHLA) und 20:3Δ^{11,14,17} als Substrate angeboten, wird im Falle der erfindungsgemäßen DHLA-Spezifität nur DHLA erkennbar zu Arachidonsäure umgesetzt.

Weiter betrifft die Erfindung rekombinante Nukleinsäuremoleküle, die folgende Elemente umfassen:
a) regulatorische Sequenzen eines in dem Zielorganismus aktiven Promotors;
b) operativ daran gebunden eine DNA-Sequenz, die ein Protein mit der enzymatischen Aktivität einer für Dihomo-γ-Linolensäure spezifischen Δ5-Desaturase kodiert;
c) ggf. operativ daran gebunden regulatorische Sequenzen, die in dem Zielorganismus als Transkriptions-Terminations- und/oder Polyadenylierungssignale dienen können.

Bevorzugt handelt es sich bei der kodierenden DNA-Sequenz um die in SEQ ID NO. 1 angegebene Sequenz aus *Phytophthora megasperma.* Der Zielorganismus ist bevorzugt eine Pflanze bzw. Pflanzenzelle oder eine Hefezelle. Aber auch andere Organismen, wie z.B. Pilze, Algen kommen in Frage.

Die in SEQ ID No. 1 angegebene Nukleinsäuresequenz weist zu den aus dem Stand der Technik bekannten Δ5-Desaturasen aus Dictyostelium discoideum (58%) und aus Mortierella alpina (54%) sowie zu Δ6-Desaturase aus Synchocystis sp. (46%) und Homo sapiens (43%) Sequenzhomologien auf, aus denen der Fachmann keine Unterscheidung zwischen Δ5- bzw. Δ6-Acyl-Lipid- oder sogar Δ8-Sphingolipid-Denaturasen hätte treffen können (die Bestimmung der Sequenzähnlichkeit erfolgte mit dem Alignment-Programm HUSAR BLAST X2).

Die DNA-Sequenz, die ein Protein mit der enzymatischen Aktivität einer für Dihomo-γ-Linolensäure spezifischen Δ5-Desaturase kodiert, kann aus natürlichen Quellen isoliert oder nach herkömmlichen Verfahren synthetisiert werden. Mittels gängiger molekularbiologischer Techniken (siehe beispielsweise Sambrook et al. (1989) Molecular Cloning: A Laboratory Manual, 2. Auflage, Cold Spring Harbor Laboratory Press, Cold Spring Harbor, New York) ist es möglich, gewünschte Konstrukte für die Transformation von Pflanzenzellen bzw. Hefezellen vorzubereiten bzw. herzustellen. Die für die gentechnische Manipulation in prokaryontischen Zellen üblicherweise eingesetzten Klonierungs-, Mutagenisierungs-, Sequenzanalyse-, Restriktionsanalyse- und weitere biochemisch-molekularbiologische Methoden sind dem Durchschnittsfachmann wohl bekannt. So können nicht nur geeignete chimäre Genkonstrukte mit der gewünschten Fusion von Promotor und erfindungsgemäßer Δ5-Desaturase-DNA-Sequenz und ggf. weiteren Regulations- und/oder Signalsequenzen hergestellt werden, vielmehr kann der Fachmann, falls erwünscht, zusätzlich mittels Routinetechniken, verschiedenartige Mutationen in die erfindungsgemäße Δ5-Desaturase kodierende DNA-Sequenz einführen, wodurch es zur Synthese von Proteinen mit evtl. veränderten biologischen Eigenschaften kommt. Hierbei ist zum einen die Erzeugung von Deletionsmutanten möglich, bei denen durch fortschreitende Deletion vom 5'- oder vom 3'-Ende der kodierenden DNA-Sequenz die Synthese entsprechend verkürzter Proteine erreicht werden kann. Ferner ist es möglich, gezielt Enzyme herzustellen, die durch Addition entsprechender Signalsequenzen in bestimmten Kompartimenten der Pflanzen- oder Hefezelle lokalisiert sind. Derartige Sequenzen sind in der Literatur beschrieben und dem Durchschnittsfachmann wohlbekannt. Weiterhin ist auch die Einführung von Punktmutationen an Positionen denkbar, bei denen eine Veränderung der Aminosäuresequenz einen Einfluß beispielsweise auf die Enzymaktivität oder die Regulierung des Enzyms hat. Auf diese Weise können z.B. Mutanten hergestellt werden, die nicht mehr den normalerweise in der Zelle herrschenden Regulationsmechanismen über allosterische Regulation oder kovalente Modifizierung unterliegen. Des weiteren können Mutanten hergestellt werden, die eine veränderte Substrat- oder Produktspezifität aufweisen. Weiterhin können Mutanten hergestellt werden, die ein verändertes Aktivitäts-, Temperatur- und/oder pH-Profil aufweisen.

Für die gentechnische Manipulation in prokaryontischen Zellen können die erfindungsgemäßen rekombinanten Nukleinsäuremoleküle oder Teile davon in Plasmide eingebracht werden, die eine Mutagenese oder eine Sequenzveränderung durch Rekombination von DNA-Sequenzen erlauben. Mit Hilfe von Standardverfahren (siehe z.B. Sambrook et al. (1989), supra) können Basenaustausche vorgenommen oder natürliche oder synthetische Sequenzen hinzugefügt werden. Für die Verbindung der DNA-Fragmente untereinander können an die Fragmente, wo erforderlich, Adapter oder Linker angefügt werden. Ferner können mittels enzymatischer und anderer Manipulationen passende Restriktionsschnittstellen zur Verfügung gestellt oder überflüssige DNA oder Restriktionsschnittstellen entfernt werden. Wo Insertionen, Deletionen oder Substitutionen in Frage kommen, können in vitro-Mutagenese, "primer repair", Restriktion oder Ligation verwendet werden. Als Analysemethoden werden im allgemeinen Sequenzanalyse, Restriktionsanalyse und weitere biochemisch-molekularbiologische Methoden durchgeführt.

In einer bevorzugten Ausführungsform ist die DNA-Sequenz, die ein Protein mit der enzymatischen Aktivität einer für Dihomo-γ-Linolensäure spezifischen Δ5-Desaturase kodiert, ausgewählt aus der Gruppe, bestehend aus
a) DNA-Sequenzen, die eine Nukleotidsequenz umfassen, die die in SEQ ID No. 2 angegebene Aminosäuresequenz oder Fragmente davon kodieren, wobei die Länge der Fragmente ausreicht, um enzymatisch aktiv zu sein;
b) DNA-Sequenzen, die die in SEQ ID No. 1 angegebene kodierende Nukleotidsequenz oder Fragmente davon umfassen, wobei die Länge der Fragmente ausreicht, um ein enzymatisch aktives Protein zu kodieren;
c) DNA-Sequenzen, die eine Nukleotidsequenz, die mit einem komplementären Strang der Nukleotidsequenz von a) oder b) hybridisieren, oder Fragmente dieser Nukleotidsequenz umfassen, wobei die Länge der Fragmente ausreicht, um ein enzymatisch aktives Protein zu kodieren;
d) DNA-Sequenzen, die eine Nukleotidsequenz, die zu einer Nukleotidsequenz von c) degeneriert ist, oder Fragmente dieser Nukleotidsequenz umfassen, wobei die Länge der Fragmente ausreicht, um ein enzymatisch aktives Protein zu kodieren;
e) DNA-Sequenzen, die ein Derivat, Analog oder Fragment einer Nukleotidsequenz von a), b), c) oder d) darstellen, wobei die Länge des Fragments ausreicht, um ein enzymatisch aktives Protein zu kodieren.

Der Begriff "Hybridisierung" bedeutet im Zusammenhang dieser Erfindung eine Hybridisierung unter konventionellen Hybridisierungsbedingungen, vorzugsweise unter stringenten Bedingungen, wie sie beispielsweise in Sambrook et al. (1989, vide supra) beschrieben sind.

Erfindungsgemäß wird eine Hybridisierung in vitro immer unter Bedingungen durchgeführt werden, die stringent genug sind, um eine spezifische Hybridisierung zu gewährleisten. Solche stringenten Hybridisierungsbedingungen sind dem Fachmann bekannt und können der Literatur entnommen werden (Sambrook et al., 2001, Molecular cloning: A laboratory manual, 3rd edition, Cold Spring Harbor Laboratory Press).

Allgemein bedeutet "spezifisch hybridisieren", dass ein Molekül unter stringenten Bedingungen präferenziell an eine bestimmte Nukleotidsequenz bindet, wenn diese Sequenz in einem komplexen Gemisch von (z.B. Gesamt-) DNA oder RNA vorliegt. Der Begriff "stringente Bedingungen" steht allgemein für Bedingungen, unter denen eine Nukleinsäuresequenz präferenziell an ihre Zielsequenz hybridisieren wird, und zu einem deutlich geringeren Ausmaß oder überhaupt nicht an andere Sequenzen. Stringente Bedingungen sind z.T. Sequenz-abhängig und werden unter verschiedenen Umständen unterschiedlich sein. Längere Sequenzen hybridisieren spezifisch bei höheren Temperaturen. Im Allgemeinen werden stringente Bedingungen so ausgewählt, dass die Temperatur etwa 5 °C unter dem thermischen Schmelzpunkt (Tₘ) für die spezifische Sequenz bei einer definierten Ionenstärke und einem definierten pH liegt. Die Tₘ ist die Temperatur (unter definierter Ionenstärke, pH und Nukleinsäurekonzentration), bei der 50% der zu der Zielsequenz komplementären Moleküle zu der Zielsequenz im Gleichgewichtszustand hybridisieren. Typischerweise sind stringente Bedingungen solche, bei denen die Salzkonzentration mindestens ungefähr 0,01 bis 1,0 M Natriumionen-Konzentration (oder ein anderes Salz) bei einem pH zwischen 7,0 und 8,3 beträgt und die Temperature mindestens ungefähr 30 °C für kurze Moleküle (also z.B. 10-50 Nukleotide) beträgt. Zusätzlich können stringente Bedingungen, wie oben bereits erwähnt, durch Zugabe destabilisierender Agenzien, wie beispielsweise Formamid, erreicht werden.

DNA-Sequenzen, die mit den DNA-Sequenzen, die ein Protein mit der enzymatischen Aktivität einer Δ5-Desaturase mit Spezifität für Dihomo-γ-Linolensäure kodieren, hybridisieren, können z.B. aus genomischen oder cDNA-Bibliotheken eines beliebigen Organismus, der die erfindungsgemäßen Δ5-Desaturase-DNA-Sequenzen natürlicherweise besitzt, isoliert werden. Die Identifizierung und Isolierung derartiger DNA-Sequenzen kann dabei z.B. unter Verwendung von DNA-Sequenzen erfolgen, die exakt oder im wesentlichen die in SEQ ID No. 1 angegebene Nukleotidsequenz oder Teile davon aufweisen, bzw. der reversen Komplemente dieser DNA-Sequenzen, z.B. mittels Hybridisierung nach Standardverfahren (siehe z.B. Sambrook et al. (1989), vide supra). Bei den als Hybridisierungssonde verwendeten Fragmenten kann es sich auch um synthetische Fragmente handeln, die mit Hilfe üblicher Synthesetechniken hergestellt wurden und deren Sequenz im wesentlichen mit einer der oben erwähnten Δ5-Desaturase-DNA-Sequenzen oder einem Teil davon übereinstimmt. Selbstverständlich können erfindungsgemäße DNA-Sequenzen auch mittels anderer Verfahren, wie z.B. PCR, isoliert werden.

Die DNA-Sequenzen, die ein Protein mit der biologischen Aktivität einer Δ5-Desaturase mit Spezifität für Dihomo-γ-Linolensäure kodieren, umfassen auch DNA-Sequenzen, deren Nukleotidsequenzen zu der einer der vorstehend beschriebenen DNA-Sequenzen degeneriert ist. Die Degeneration des genetischen Codes bietet dem Fachmann u.a. die Möglichkeit, die Nukleotidsequenz der DNA-Sequenz an die Codonpräferenz (codon usage) des Zielorganismus, also der aufgrund der Expression der erfindungsgemäßen Enzymaktivität einen veränderten Gehalt an Arachidonsäure aufweisenden Pflanze bzw. Pflanzenzelle oder Hefezelle anzupassen und die Expression dadurch zu optimieren.

Die oben beschriebenen DNA-Sequenzen umfassen auch Fragmente, Derivate und allelische Varianten der oben beschriebenen DNA-Sequenzen, die ein Protein mit der biologischen Aktivität einer für Dihomo-γ-Linolensäure spezifischen Δ5-Desaturase kodieren. Unter "Fragmenten" werden dabei Teile der DNA-Sequenz verstanden, die lang genug sind, um eines der beschriebenen Proteine zu kodieren. Der Ausdruck "Derivat" bedeutet in diesem Zusammenhang, dass die Sequenzen sich von den oben beschriebenen DNA-Sequenzen an einer oder mehreren Positionen unterscheiden, aber einen hohen Grad an Homologie zu diesen Sequenzen aufweisen.

Homologie bedeutet dabei eine Sequenzidentität von mindestens 60 Prozent, 64 Prozent, 68 Prozent, insbesondere eine Identität von mindestens 70 Prozent, 72 Prozent, 74 Prozent, 76 Prozent, 78 Prozent, vorzugsweise von mindestens 80 Prozent, 82 Prozent, 84 Prozent, 86 Prozent, 88 Prozent, besonders bevorzugt von mindestens 90 Prozent, 92 Prozent, 94 Prozent und am meisten bevorzugt von mindestens 95 Prozent, 97 Prozent, 99 Prozent.

Dabei weisen die durch diese DNA-Sequenzen kodierten Enzyme eine Sequenzidentität zu der in SEQ ID No. 2 angegebenen Aminosäuresequenz von mindestens 60, 64, 68, 70, 74, 78 Prozent, insbesondere mindestens 80, 82 Prozent, vorzugsweise mindestens 84, 86, 88, 90 Prozent und besonders bevorzugt von mindestens 92, 94, 96, 98 Prozent auf. Die Abweichungen zu den oben beschriebenen DNA-Sequenzen können dabei beispielsweise durch Deletion, Substitution, Insertion oder Rekombination entstanden sein.

Homologiegrade bzw. Sequenzidentitäten werden üblicherweise über verschiedene Alignment-Programme, wie z. B. CLUSTAL festgestellt. Allgemein stehen dem Fachmann zur Bestimmung der Sequenzidentität/-ähnlichkeit geeignete Algorithmen zur Verfügung, z.B. auch das Programm, das unter http://www.ncbi.nlm.nih.gov/BLAST (z.B. der Link "Standard nucleotide-nucleotide BLAST [blastn]") zugänglich ist.

Bei den DNA-Sequenzen, die homolog zu den oben beschriebenen Sequenzen sind und Derivate dieser Sequenzen darstellen, handelt es sich in der Regel um Variationen dieser Sequenzen, die Modifikationen darstellen, die dieselbe biologische Funktion ausüben. Es kann sich dabei sowohl um natürlicherweise auftretende Variationen handeln, beispielsweise um Sequenzen aus anderen Organismen oder um Mutationen, wobei diese Mutationen auf natürliche Weise aufgetreten sein können oder durch gezielte Mutagenese eingeführt wurden. Ferner kann es sich bei den Variationen um synthetisch hergestellte Sequenzen handeln. Bei den allelischen Varianten kann es sich sowohl um natürlich auftretende Varianten handeln, als auch um synthetisch hergestellte oder durch rekombinante DNA-Techniken erzeugte Varianten.

In einer besonders bevorzugten Ausführungsform stammt die beschriebene, für eine Δ5-Desaturase mit Dihomo-γ-Linolensäure-Spezifität kodierende DNA-Sequenz aus *Phytophthora megasperma.*

Weiter betrifft die Erfindung Nukleinsäuremoleküle, die die erfindungsgemäßen Nukleinsäuresequenzen enthalten oder durch natürlich vorkommende oder durch gentechnische oder chemische Prozesse und Syntheseverfahren aus diesen entstanden sind bzw. von diesen abgeleitet wurden. Hierbei kann es sich beispielsweise um DNA- oder RNA-Moleküle, cDNA, genomische DNA, mRNA usw. handeln.

Die Erfindung betrifft ebenfalls solche Nukleinsäuremoleküle, in denen die erfindungsgemäßen Nukleinsäuresequenzen mit regulatorischen Elementen verknüpft sind, die die Transkription und, falls erwünscht, die Translation in der transgenen Zelle gewährleisten.

Für die Expression der in den erfindungsgemäßen rekombinanten Nukleinsäuremolekülen enthaltenen DNA-Sequenzen in pflanzlichen Zellen kommt grundsätzlich jeder in pflanzlichen Zellen aktive Promotor in Frage. So können die erfindungsgemäßen DNA-Sequenzen beispielsweise unter Kontrolle konstitutiver, aber auch induzierbarer oder gewebe- bzw. entwicklungsspezifischer Regulationselemente, insbesondere Promotoren, in Pflanzenzellen exprimiert werden. Während beispielsweise die Verwendung eines induzierbaren Promotors die gezielt ausgelöste Expression der erfindungsgemäßen DNA-Sequenzen in Pflanzenzellen ermöglicht, bietet beispielsweise der Einsatz von gewebespezifischen, beispielsweise blatt- oder samenspezifischen, Promotoren die Möglichkeit, den Gehalt an Arachidonsäure in bestimmtem Gewebe, z.B. in Blatt- bzw. Samengewebe, zu verändern. Andere geeignete Promotoren vermitteln z.B. Licht-induzierte Genexpression in transgenen Pflanzen. In Bezug auf die zu transformierende Pflanze kann der Promotor homolog oder heterolog sein.

Geeignete Promotoren sind z.B. der 35S RNA-Promotor des Cauliflower Mosaic Virus und der Ubiquitin-Promotor aus Mais für eine konstitutive Expression. Als samenspezifische Promotoren bieten sich bspw. der USP- (Bäumlein et al. (1991), Mol. Gen. Genet. 225: 459-467) oder der Hordein-Promotor (Brandt et al. (1985), Carlsberg Res. Commun. 50: 333-345) an.

Konstitutive keimungsspezifische und samenspezifische Promotoren werden im Rahmen dieser Erfindung bevorzugt, da sie sich besonders für die gezielte Erhöhung des Gehalts an Arachidonsäure in transgenen Samen eignen.

In jedem Fall kann der Fachmann geeignete Promotoren der Literatur entnehmen oder mittels Routineverfahren selbst aus beliebigen Pflanzen isolieren.

Dies gilt auch für die Expression der erfindungsgemäßen DNA-Sequenzen in Hefezellen. Hier bieten sich vor allem induzierbare Promotoren oder bspw. der OLE1-Promotor an.

Ferner sind Transkriptions- bzw. Terminationssequenzen vorhanden, die der korrekten Beendigung der Transkription dienen, sowie der Addition eines polyA-Tails an das Transkript dienen kann, dem eine Funktion bei der Stabilisierung der Transkripte beigemessen wird. Derartige Elemente sind in der Literatur beschrieben (z.B. Gielen (1989) EMBO J. 8:23-29) und beliebig austauschbar, z.B. der Terminator des Octopinsynthasegens aus *Agrobacterium tumefaciens.*

Die Erfindung betrifft weiterhin Proteine mit der biologischen Aktivität einer Δ5-Desaturase mit Spezifität für Dihomo-γ-Linolensäure oder biologisch aktive Fragmente davon, die durch eine erfindungsgemäße Nukleinsäuresequenz oder ein erfindungsgemäßes Nukleinsäuremolekül kodiert werden. Vorzugsweise handelt es sich um eine pilzliche Δ5-Desaturase, besonders bevorzugt um ein Protein mit der in SEQ ID NO. 2 gezeigten Aminosäuresequenz oder einem aktiven Fragment davon.

Eine weitere Aufgabe der Erfindung besteht darin, Vektoren bereitzustellen, deren Verwendung die Herstellung neuer Pflanzen bzw. pflanzlicher Zell- oder Gewebekulturen bzw. Hefen, in denen ein veränderter Gehalt an Arachidonsäuren erzielt werden kann, ermöglicht. Diese Aufgabe wird durch die Bereitstellung der erfindungsgemäßen Vektoren gelöst, die für Enzyme mit der Aktivität einer erfindungsgemäßen Δ5-Desaturase kodierende Nukleinsäuresequenzen enthalten.

Die vorliegende Erfindung betrifft somit auch Vektoren, insbesondere Plasmide, Kosmide, Viren, Bakteriophagen und andere in der Gentechnik gängige Vektoren, die die vorstehend beschriebenen erfindungsgemäßen Nukleinsäuremoleküle enthalten und ggf. für den Transfer der erfindungsgemäßen Nukleinsäuremoleküle auf Pflanzen bzw. Pflanzenzellen oder Hefezellen eingesetzt werden können.

Gegebenenfalls können die Nukleinsäuresequenzen der Erfindung durch Enhancer-Sequenzen oder andere regulatorische Sequenzen ergänzt sein. Diese regulatorischen Sequenzen beinhalten beispielsweise auch Signalsequenzen, die für den Transport des Genprodukts zu einem bestimmten Kompartiment sorgen.

Es ist ebenso eine Aufgabe der Erfindung, neue transgene Pflanzen, Pflanzenzellen, Pflanzenteile, transgenes Vermehrungsmaterial und transgene Ernteprodukte bereitzustellen, die sich durch eine gegenüber Wildtyppflanzen bzw. -zellen veränderten Gehalt an Arachidonsäure auszeichnen.

Diese Aufgabe wird durch die Übertragung der erfindungsgemäßen Nukleinsäuremoleküle und ihre Expression in Pflanzen gelöst. Durch die Bereitstellung der erfindungsgemäßen Nukleinsäuremoleküle besteht nun die Möglichkeit, pflanzliche Zellen mittels gentechnischer Methoden dahingehend zu verändern, dass sie im Vergleich zu Wildtypzellen eine neue oder veränderte Δ5-Desaturase-Aktivität mit Spezifität für Dihomo-γ-Linolensäure aufweisen und es als Folge davon zu einer Veränderung des Gehalts an Arachidonsäure kommt.

So betrifft die Erfindung in einer Ausführungsform Pflanzen bzw. deren Zellen und Teile, in denen der Gehalt an Arachidonsäure aufgrund der Gegenwart und Expression der erfindungsgemäßen Nukleinsäuremoleküle gegenüber Wildtyppflanzen erhöht ist.

Gegenstand der Erfindung sind weiterhin transgene Pflanzenzellen bzw. solche Pflanzenzellen umfassende Pflanzen und deren Teile und Produkte, in denen die erfindungsgemäßen Nukleinsäuremoleküle integriert in das pflanzliche Genom vorliegen. Ebenfalls Gegenstand der Erfindung sind Pflanzen, in deren Zellen die erfindungsgemäße Nukleinsäuresequenz in replizierter Form vorliegt, d.h. die Pflanzenzelle enthält die fremde DNA auf einem eigenständigen Nukleinsäuremolekül (transiente Expression).

Bei den Pflanzen, die mit den erfindungsgemäßen Nukleinsäuremolekülen transformiert sind und in denen aufgrund der Einführung eines solchen Moleküls eine veränderte Menge an Arachidonsäure synthetisiert wird, kann es sich im Prinzip um jede beliebige Pflanze handeln. Vorzugsweise ist es eine monokotyle oder dikotyle Nutzpflanze.

Beispiele für monokotyle Pflanzen sind die Pflanzen, die zu den Gattungen Avena (Hafer), Triticum (Weizen), Secale (Roggen), Hordeum (Gerste), Oryza (Reis), Panicum, Pennisetum, Setaria, Sorghum (Hirse), Zea (Mais) gehören. Bei den dikotylen Nutzpflanzen sind u.a. zu nennen Leguminosen, wie Hülsenfrüchte und insbesondere Alfalfa, Sojabohne, Raps, Tomate, Zuckerrübe, Kartoffel, Zierpflanzen oder Bäume. Weitere Nutzpflanzen können beispielsweise Obst (insbesondere Äpfel, Birnen, Kirschen, Weintrauben, Citrus, Ananas und Bananen), Ölpalmen, Tee-, Kakao- und Kaffeesträucher, Tabak, Sisal, Baumwolle, Lein, Sonnenblume sowie Heilpflanzen und Weidegräser sowie Futterpflanzen sein. Besonders bevorzugt sind die Getreide Weizen, Roggen, Hafer, Gerste, Reis, Mais und Hirse, Futtergetreide, Zuckerrübe, Raps, Soja, Tomate, Kartoffel, Süßgräser, Futtergräser und Klee.

Es ergibt sich von selbst, dass die Erfindung insbesondere übliche Nahrungs- bzw. Futterpflanzen betrifft. Hier sind neben den bereits erwähnten Pflanzen zusätzlich Erdnuß, Linse, Ackerbohne, Runkelrübe, Buchweizen, Möhre, Sonnenblume, Topinambur, Rübsen, Weißer Senf, Kohlrübe und Stoppelrübe zu nennen.

Besonders bevorzugt sind Ölsaaten und hier insbesondere die sog. 18:2-Pflanzen Sojabohne, Lein und Sonnenblume.

Gegenstand der Erfindung sind ferner Vermehrungsmaterial und Ernteprodukte von erfindungsgemäßen Pflanzen, beispielsweise Samen, Früchte, Stecklinge, Knollen, Wurzelstöcke usw., sowie Teile dieser Pflanzen, wie Protoplasten, Pflanzenzellen und Kalli.

Die Erfindung betrifft aber nicht nur die Steigerung des Gehalts an Arachidonsäure in Pflanzen zwecks Erhöhung des Nährwertes für die Ernährung von Mensch und Tier. Die Erfindung betrifft vielmehr auch die Herstellung von Arachidonsäure in Zellkulturen zwecks anschließender Gewinnung der Arachidonsäure aus den transgenen Zellen. Hierbei kann es sich zum einen um pflanzliche Zellkulturen, beispielsweise Suspensionskulturen oder Kalluskulturen, aber auch um Hefekulturen handeln, die sich für die Produktion von Arachidonsäure und anschließende Gewinnung der Arachidonsäure eignen. Selbstverständlich können auch transgene Pflanzen bzw. -teile als Produktionsstätte für Arachidonsäure zwecks Gewinnung der Arachidonsäure aus den Pflanzen bzw. Pflanzenteilen dienen. Wie Pflanzen bzw. pflanzliche Kulturen können auch Hefen bzw. Hefekulturen nicht nur zur Gewinnung der Arachidonsäure aus den transgenen, Arachidonsäure produzierenden Zellen eingesetzt werden, sie eignen sich auch unmittelbar als Nahrungsmittel mit gesteigertem Nährwert.

Bei Bedarf kann den transgenen, Arachidonsäure-produzierenden Zellen Dihomo-γ-Linolensäure zugesetzt werden, um ausreichend Ausgangsmaterial für die Arachidonsäuresynthese bereitzustellen.

Für die Gewinnung der Arachidonsäure aus den transgenen Zellen, Pflanzen oder Zellkulturen eignen sich herkömmliche Verfahren, wie z.B. eine Kaltpressung, wie sie auch für Olivenöl vorgenommen wird. Bei der Gewinnung von Arachidonsäure aus Hefezellen bzw. -kulturen eignet sich auch die Wasserdampfdestillation. Der Fachmann kann geeignete Methoden dem Stand der Technik entnehmen.

Für die Kultivierung transgener Pflanzenzellen eignen sich übliche Zellkulturmethoden, die dem Fachman bekannt sind. Gleiches gilt für die Kultivierung von Hefezellen.

In einer weiteren Ausführungsform betrifft die Erfindung Wirtszellen, insbesondere prokaryontische und eukaryontische Zellen, die mit einem oben beschriebenen Nukleinsäuremolekül oder einem Vektor transformiert bzw. infiziert wurden, sowie Zellen, die von derartigen Wirtszellen abstammen und die beschriebenen Nukleinsäuremoleküle oder Vektoren enthalten. Die Wirtszellen können Bakterien, Viren, Algen, Hefe- und Pilzzellen sowie pflanzliche oder tierische Zellen sein.

Der vorliegenden Erfindung liegt außerdem die Aufgabe zugrunde, Verfahren zur Herstellung von Pflanzenzellen und Pflanzen bereitzustellen, die sich durch einen erhöhten Gehalt an Arachidonsäure auszeichnen.

Diese Aufgabe wird durch Verfahren gelöst, mit deren Hilfe die Erzeugung neuer Pflanzenzellen und Pflanzen, die aufgrund der Übertragung der erfindungsgemäßen, für Δ5-Desaturase mit Dihomo-γ-Linolensäure-Spezifität kodierenden Nukleinsäuremoleküle einen erhöhten Gehalt an Arachidonsäure aufweisen, möglich ist. Zur Erzeugung solcher neuer Pflanzenzellen und Pflanzen bieten sich verschiedene Methoden an. Zum einen können Pflanzen bzw. Pflanzenzellen mit Hilfe herkömmlicher gentechnologischer Transformationsmethoden derart verändert werden, dass die neuen Nukleinsäuremoleküle in das pflanzliche Genom integriert werden, d.h., es werden stabile Transformanten erzeugt. Zum anderen kann ein erfindungsgemäßes Nukleinsäuremolekül, dessen Anwesenheit und Expression in der Pflanzenzelle eine veränderte Biosyntheseleistung bewirkt, in der Pflanzenzelle bzw. der Pflanze als selbstreplizierendes System enthalten sein. So können die erfindungsgemäßen Nukleinsäuremoleküle beispielsweise in einem Virus enthalten sein, mit dem die Pflanze bzw. Pflanzenzelle in Kontakt kommt.

Erfindungsgemäß werden Pflanzenzellen und Pflanzen, die aufgrund der Expression einer erfindungsgemäßen Nukleinsäuresequenz einen erhöhten Gehalt an Arachidonsäure aufweisen, durch ein Verfahren hergestellt, das folgende Schritte umfaßt:
a) Herstellung eines rekombinanten Nukleinsäuremoleküls, umfassend die folgenden Bestandteile in 5' -> 3'-Orientierung:
   - regulatorische Sequenzen eines in Pflanzenzellen aktiven Promotors,
   - operativ daran gebunden eine Nukleinsäuresequenz, die für ein Protein mit der enzymatischen Aktivität einer Δ5-Desaturase mit Dihomo-γ-Linolensäure-Spezifiät kodiert, und
   - ggf. operativ daran gebunden Sequenzen, die als Transkriptions-, Terminations- und/oder Polyadenylierungssignale in Pflanzenzellen dienen können.
b) Übertragung des Nukleinsäuremoleküls aus a) auf pflanzliche Zellen.

Im Falle von Hefezellen wird das oben genannte Verfahren dahingehend modifiziert, dass regulatorische Sequenzen eines in Hefezellen aktiven Promotors bzw. entsprechende Transkriptions-, Terminations- und/oder Polyadenylierungssignale verwendet werden.

Zur Vorbereitung der Einführung fremder Gene in höhere Pflanzen bzw. deren Zellen stehen eine große Anzahl von Klonierungsvektoren zur Verfügung, die ein Replikationssignal für *E*. *coli* und ein Markergen zur Selektion transformierter Bakterienzellen enthalten. Beispiele für derartige Vektoren sind pBR322, pUC-Serien, M13mp-Serien, pACYC184 usw. Die gewünschte Sequenz kann an einer passenden Restriktionsschnittstelle in den Vektor eingeführt werden. Das erhaltene Plasmid wird dann für die Transformation von *E. coli*-Zellen verwendet. Transformierte *E*. *coli*-Zellen werden in einem geeigneten Medium gezüchtet und anschließend geerntet und lysiert, und das Plasmid wird wiedergewonnen. Als Analysenmethode zur Charakterisierung der gewonnenen Plasmid-DNA werden im allgemeinen Restriktionsanalysen, Gelelektrophoresen und weitere biochemisch-molekularbiologische Methoden eingesetzt. Nach jeder Manipulation kann die Plasmid-DNA gespalten und gewonnene DNA-Fragmente mit anderen DNA-Sequenzen verknüpft werden.

Für die Einführung von DNA in eine pflanzliche Wirtszelle stehen eine Vielzahl von Techniken zur Verfügung, wobei der Fachmann die jeweils geeignete Methode ohne Schwierigkeiten ermitteln kann. Diese Techniken umfassen die Transformation pflanzlicher Zellen mit T-DNA unter Verwendung von *Agrobacterium tumefaciens* oder *Agrobacterium rhizogenes* als Transformationsmedium, Diffusion von Protoplasten, die Injektion, die Elektroporation, den direkten Gentransfer isolierter DNA in Protoplasten, die Einbringung von DNA mittels biolistischer Methoden sowie weitere Möglichkeiten, die bereits seit mehreren Jahren gut etabliert sind und zum üblichen Repertoire des Fachmanns in der pflanzlichen Molekularbiologie bzw. Pflanzenbiotechnologie und Zell- und Gewebekultur gehören und in allseits bekannten Übersichtsartikeln und Handbüchern zur Pflanzentransformation beschrieben sind.

Ist die eingeführte DNA einmal im Genom der Pflanzenzelle integriert, so ist sie dort in der Regel stabil und bleibt auch in den Nachkommen der ursprünglich transformierten Zelle erhalten. Sie enthält normalerweise einen Selektionsmarker, der den transformierten Pflanzenzellen Resistenz gegenüber einem Biozid oder einem Antibiotikum wie Kanamycin, G 418, Bleomycin, Hygromycin, Methotrexat, Glyphosat, Streptomycin, Sulfonylharnstoff, Gentamycin oder Phosphinotricin u.a. vermittelt. Der individuell gewählte Marker sollte daher die Selektion transformierter Zellen gegenüber Zellen, denen die eingeführte DNA fehlt, gestatten. Hierzu sind auch alternative Marker geeignet, wie nutritive Marker, Screeningmarker (wie GFP, green fluorescent protein). Selbstverständlich kann auch vollkommen auf Selektionsmarker verzichtet werden, was allerdings mit einem ziemlich hohen Screeningbedarf einhergeht. Falls der eingesetzte Selektionsmarker nach erfolgter Transformation und Identifizierung erfolgreich transformierter Zellen bzw. Pflanzen wieder entfernt werden soll, stehen dem Fachmann hierfür verschiedene Strategien zur Verfügung. So können z.B. sequenzspezifische Rekombinasen verwendet werden, z.B. in Form der Retransformation einer Rekombinase-exprimierenden Ausgangslinie und Auskreuzung der Rekombinase nach erfolgter Entfernung des Selektionsmarkers. Der Selektionsmarker kann auch durch Cotransformation mit anschließender Auskreuzung entfernt werden.

Die Regeneration der transgenen Pflanzen aus transgenen Pflanzenzellen erfolgt, falls erwünscht, nach üblichen Regenerationsmethoden unter Verwendung üblicher Nährmedien und Phytohormone. Die so erhaltenen Pflanzen können dann, falls erwünscht, mittels üblicher Verfahren, einschließlich molekularbiologischer Methoden, wie PCR, Blot-Analysen, oder biochemischer Verfahren auf Anwesenheit der eingeführten DNA, die ein Protein mit der enzymatischen Aktivität einer Dihomo-γ-Linolensäure-spezifischen Δ5-Desaturase kodiert, bzw. auf Anwesenheit von Δ5-Desaturase-Enzymaktivität untersucht werden.

Selbstverständlich können Pflanzenzellen, die die erfindungsgemäßen Nukleinsäuremoleküle enthalten, auch als Pflanzenzellen (einschließlich Protoplasten, Kalli, Suspensionskulturen und dergleichen) weiterkultiviert werden. Die Erfindung betrifft auch die Herstellung von Arachidonsäure in pflanzlichen Kulturen.

Erfindungsgemäß umfasst der Begriff transgene Pflanze sowohl die Pflanze in ihrer Gesamtheit, als auch alle transgenen Pflanzenteile, in denen eine erfindungsgemäße Desaturase exprimiert wird. Bei solchen Pflanzenteilen kann es sich beispielsweise um Pflanzenzellen handeln, um Pflanzensamen, um Blätter, um Blüten, um Früchte, um Speicherorgane wie Knollen und um Pollen. Mit "transgener Pflanze" ist erfindungsgemäß auch das Vermehrungsmaterial von erfindungsgemäßen transgenen Pflanzen gemeint wie z. B. Samen, Früchte, Stecklinge, Knollen, Wurzelstücke etc., wobei dieses Vermehrungsmaterial ggf. oben beschriebene transgene Pflanzenzellen enthält, sowie transgene Teile dieser transgenen Pflanzen wie Protoplasten, Pflanzenzellen und Kalli.

Die Transformation, Expression und Kultivierung der Hefezellen kann nach herkömmlichen Protokollen durchgeführt werden, z.B. wie beschrieben in Ausubel et al. (2000) Current Protokols in Molecular Biology. Beispiele hierfür sind auch in den unten stehenden Ausführungsbeispielen angegeben.

Ferner betrifft die Erfindung ein Verfahren zur Gewinnung von Arachidonsäure aus Pflanzen- bzw. Hefezellen. Hierzu stehen dem Fachmann in der Literatur Protokolle zur Reinigung von C20-Fettsäuren und zur Reinigung von Arachidonsäure im Besonderen zur Verfügung.

Die nachfolgenden Beispiele dienen zur Erläuterung der Erfindung, ohne diese in irgendeiner Weise einzuschränken.

### Beispiele

Isolierung der für eine Δ5-Desaturase aus *Phytophthora megasperma* kodierenden cDNA

Aus 2 Tage alten *Phytophthtora megasperma-Myzelien,* die auf einem Oleatenthaltenden Medium kultiviert wurden, wurde poly(A)⁺-RNA isoliert (Oligotex-dT mRNA Maxi Kit, Firma Qiagen, Hilden, Deutschland). Anschließend wurde unter Einsatz von 5 µg der poly(A)⁺-RNA die cDNA-Synthese mit dem Superscript^{™} cDNA-Synthese-Kit der Firma Lifetechnologies (Eggenstein, Deutschland) durchgeführt. Die erhaltene cDNA besitzt asymmetrische Enden. Anschließend erfolgte die Ligation der cDNA in das Plasmid pSPORT-1 (Lifetechnologies, Eggenstein, Deutschland) , das zuvor mit den Restriktionsenzymen SalI und NotI geschnitten worden war. Die Ligation erfolgte mit T4-Ligase nach dem Herstellerprotokoll (Lifetechnologies).

Anschließend wurden Zellen des *E*. *coli*-Stammes XL1-Blue mit dem Ligationsansatz, als mit in pSPORT-1 ligierter cDNA, mittels Elektroporation transformiert und auf LB-Platten mit Ampicillin ausplattiert, so dass pro Agarplatte mit 15 cm Durchmesser 10000 bis 15000 Kolonien wuchsen.

Von den Agarplatten wurden die Kolonien auf je 2 Nitrocellulosefilter transferiert und die Hybridisierung durchgeführt. Die Hybridisierung erfolgte nach dem Stand der Technik (Sambrook et al., 1989, supra) mit α-[³²P]-dCTP markierten Nukleotidsequenzen. Kolonien, die ein Hybridisierungssignal zeigten, wurden vermehrt und sequenziert.

Die cDNA-Bank wurde mit folgenden Nukleotidsequenzen gescreent:
Oligo 1 5'-GTG GCC AAG CAC AAC ACG GCC AAG AGC-3'
Oligo 2 5'-ACC ATC CGC GGC GTC GTC TAC GAC GTG ACC-3'

Ein positiver cDNA-Klon ist in SEQ ID No. 1 angegeben. Diese DNA-Sequenz aus *P. megasperma* kodiert für ein Enzym mit der Aktivität einer Δ5-Desaturase mit 20:3-Substratspezifität.

### Expression des Δ5-Desaturase-cDNA-Klons in Hefe

Zunächst wurde der cDNA-Klon in den Hefe-Expressionsvektor pYES2 (Invitrogen, Groningen, Niederlande) umkloniert. Dies erfolgte unter Einsatz der folgenden Oligonukleotidprimer:
5'-GGA TCC ATG GCC CCC ATC GAG ACT GTC AAA G3' (Primer a)
   und
5'-GCA TGC CCC GCG TTA TCC AGC CAA AGC TTA CC3' (Primer b)

Der Primer a enthält eine Restriktionsschnittstelle für BamHI (Unterstreichung), der Primer b enthält eine Schnittstelle für das Restriktionsenzym SphIb (Unterstreichung).

Die PCR wurde nach folgendem PCR-Protokoll durchgeführt:

### PCR-Reaktionsansatz:

| | | |
|---|---|---|
| dNTP-Mix | 1 µl | |
| 5'-Primer (Primer a) | 4 µl | |
| 3'-Primer (Primer b) | 4 µl | |
| Template | 1 µl | Plasmid-DNA (10 ng) |
| Polymerase | 0,5 µl | High fidelity, Roche Diagnostics GmbH |
| 10 x Puffer | 5 µl | |
| Wasser | 34,5 µl | |
| Gesamtvolumen | 50 µl | |

Die PCR wurde unter folgenden Bedingungen durchgeführt:
- 2 Min. 94°C
- 10 Zyklen von: 30 Sekunden bei 94°C, 30 Sekunden bei 55°C, 1 Minute bei 72°C
- 15 Zyklen von Zeitinkrement: 5 Sekunden: 30 Sekunden bei 94°C, 30 Sekunden bei 55°C, 1 Minute bei 72°C
- 5 Minuten bei 72°C

Anschließend wurde das erhaltene PCR-Fragment in den Vektor pGEM-T (Promega, Madison, USA) zur Zwischenklonierung ligiert. Der Ligationsansatz wurde in XL1-Blue-Zellen transformiert.

Die aus den transformierten Zellen erhaltene Minipräp-DNA-(aus 5 ml Übernachtkultur, 37°C, Aufarbeitung durchgeführt mit Spin-Prep-Kit von Macherey & Nagel, Düren, Deutschland) wurde mit den Restriktionsenzymen BamHI und PaeI von MBI Fermentas (St. Leon-Rot, Deutschland) verdaut.

Anschließend erfolgte die Ligation mittels T4-Ligase von MBI Fermentas in den Hefe-Expressionsvektor pYES2, der zuvor ebenfalls mit den Restriktionsenzymen BamHI und PaeI verdaut worden war. Der Ligationsansatz wurde in *E*. *coli-*XL1-Blue-Zellen transformiert.

5 ml-Übernachtkulturen von verschiedenen Klonen wurden hinsichtlich ihrer Plasmid-DNA analysiert (Minipräp-DNA-Isolierung mit dem Spin-Prep Kit von Macherey & Nagel). Der gewünschte Klon (in sense-Orientierung hinter dem Galaktose-Promotor Gall) wurde in den Hefestamm INVSc1 (Invitrogen, Groningen, Niederlande) mit der Litiumacetat-Methode transformiert (Ausubel et al. (2000) supra).

Die transformierten Hefezellen wurden bei 30°C in SD-Medium (Ausubel et al. (2000) supra), enthaltend Glucose (2%) und Aminosäurelösung, ohne Uracil, über Nacht kultiviert. Anschließend wurde eine Hauptkultur nach zweimaligem Waschen der Vorkultur ohne Zucker in SD-Medium, enthaltend Galactose und Aminosäurelösung, ohne Uracil, und enthaltend Linolsäure (20 mg, 0,02%) oder Dihomo-γ-Linolensäure (15 mg, 0,015%) und Tergitol (10%) bei 30°C für 72 Stunden kultiviert.

Diese Hauptkultur wurde anschließend durch Zentrifugation, Gefriertrocknung der Proben und Transmethylierung der Proben mit Natriummethylat geerntet.

### Expression in Hefezellen

Alle Hefetechniken sind Stand der Technik laut Ausubel et al., 2000. Current Protocols in Molecular Biology.

### Vorkultur

20 ml SD-Medium + Glucose + Aminosäurelösung ohne die entsprechende Aminosäure für die Selektion werden mit einer Einzelkolonie beimpft, über Nacht bei 30°C mit 140 rpm inkubiert.

### Hauptkultur

Vorkultur 2 mal waschen (durch Abzentrifugieren und Resuspendieren) in SD-Medium ohne Zucker, dann die Hauptkultur animpfen auf eine OD₆₀₀ = 0,1- 0,3.

Kultivierung der Hauptkultur in SD-Medium + Galaktose + Aminosäurelösung ohne die entsprechende Aminosäure + Fettsäure, Tergitol NP40 (10%) bei 30°C für 72 h mit 140 rpm schüttelnd.

Ernten der Hauptkultur durch Zentrifugation in 50 ml sterilen Zentrifugenröhren.

### Gefriertrocknung des Hefezell-Pellets

Gefrorene Zellpellets werden für ca. 18 h lyophyllisiert.

### Transmethylierung

Trockene Probe + 1,35 ml Methanol:Toluol (2:1) + 0,5 ml Na-Methoxid-Lösung mit Glasstab möglichst fein zermörsern
1 h bei RT schüttelnd auf Belly Dancer inkubieren lassen
1,8 ml 1M NaCl-Lösung zugeben
2 ganze Pasteurpipetten n-Heptan dazu
10 min auf Belly Dancer schüttelnd extrahieren
Abzentrifugieren (10 min, 400 rpm, 4°C)
Heptan-Überstand in Reagenzglas überführen
Unter N₂ verblasen
Mit 3 x 0,3 ml Hexan in Eppi überführen
Unter N₂ verblasen und Rückstand in MeCN aufnehmen

### GC-Analytik

7 µl Probe (in MeCN) ins Probenröhrchen aufgegeben
1 µl in die GC injiziert
5 µl Probe wurden unter Stickstoff-Strom eingedampft. Der Rückstand wurde in 400 µl MeOH aufgenommen und 10 µl EDAC-Lösung (10 ml EDAC/100 µl MeOH) wurden hinzugegeben.

Anschließend wurde für 2 Stunden bei Raumtemperatur geschüttelt. Nach Zugabe von 200 µl Tris-Puffer (0,1 M, pH 7,4) wurde zweimal mit je 1 ml Hexan ausgeschüttelt. Die Hexan-Phasen wurden vereinigt und unter Stickstoff-Strom eingedampft. Der Rückstand wurde in 20 µl MeCN aufgenommen.

Die GC-Analytik erfolgte unter folgenden Bedingungen:

Säule: HP-INNOWax (cross-linked PEG), 30 m x 0,32 mm x 0,5 µm

| | |
|---|---|
| Flußrate: | 1,5 ml/min (konstanter Fluß) Helium 150°C |
| Injektion: | 220°C |
| Ofen: | 150°C (1 min), auf 200°C (15 K/min), auf 250°C (2 K/min), 250°C (5 Minuten) |
| Detektion: | FID 275°C. |

In Fütterungsexperimenten konnte eindeutig bestätigt werden, dass die erfindungsgemäßen Desaturasen hochspezifisch für DGLA sind, diese Enzyme also nur dieses Substrat nachweisbar zu Arachidonsäure umsetzen.

Im Rahmen der Fütterungsexperimente wurde der Hefestamm INVScl in der Regel für 3 Tage bei 30°C auf Medium kultiviert, das je nach Experiment verschiedene C20-Fettsäuren enthielt. Als Kontrolle diente in der Regel der Hefestamm INVSc1, der den Kontrollvektor pYES2 ohne Desaturase-Sequenz enthielt.

### Beschreibung der Abbildungen

Abb. 1 zeigt eine schematisierte PLTFA-Biosynthese in Tieren.
Abb. 2 zeigt das Ergebnis der GC-Analyse:

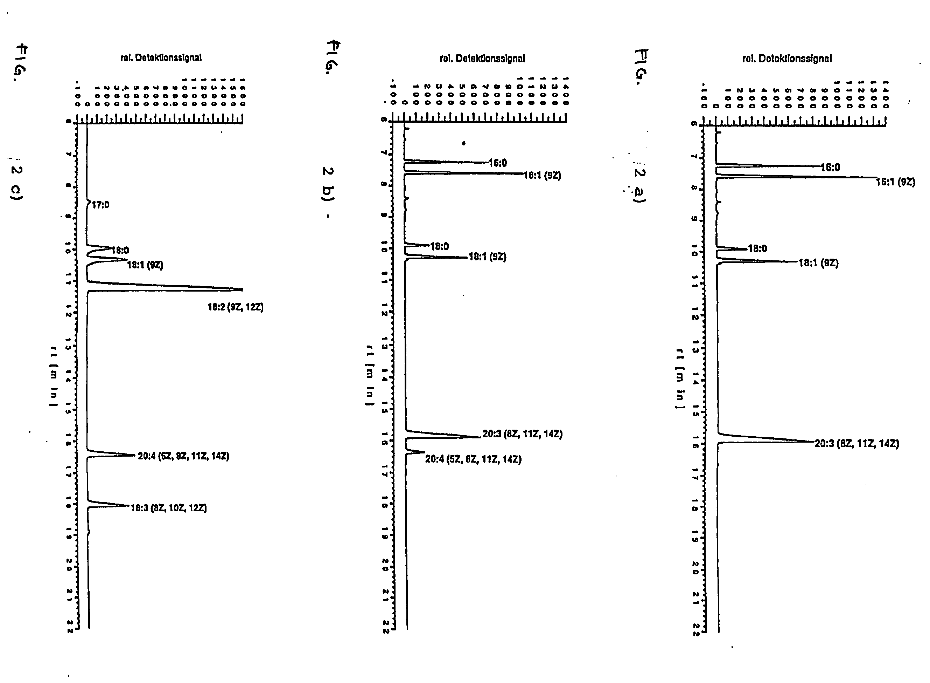

Abb. 2 a) Hefestamm INVSc1 mit pYES2 (Kontrolle) und Fütterung mit Dihomo-γ-Linolensäure (Dihomo γ-LEA);
Abb. 2 b) Hefestamm INVSc1 mit pYES2 + Δ5-Desaturase und Fütterung mit Dihomo-γ-Linolensäure;
Abb. 2 c) Standard.
Abb. 3 zeigt das Ergebnis einer GC-Analyse:

| | |
|---|---|
| Oben: | Hefestamm INVSc1 mit pYES2 (Kontrolle) und Fütterung mit Dihomo-γ-Linolensäure (Dihomo γ-LEA); |
| Mitte: | Hefestamm INVSc1 mit pYES2 + Δ5-Desaturase und Fütterung mit Dihomo-γ-Linolensäure; |
| Unten: | Standard. |

Abb. 4 zeigt das Ergebnis einer GC-Analyse eines Kofütterungsexperiments mit drei verschiedenen C20-Fettsäuren:

| | |
|---|---|
| Oben: | Hefestamm INVScl mit pYES2 (Kontrolle) und Fütterung mit Dihomo-γ-Linolensäure (Dihomo γ-LEA, 20:3Δ^{8,11,14}), 20:2Δ^{13,16} und 20:3Δ^{11,14,17}; |
| Mitte: | Hefestamm INVSc1 mit pYES2 + Δ5-Desaturase und Fütterung mit Dihomo-γ-Linolensäure; |
| Unten: | Standard. |

Als Produkt ist nur Arachidonsäure nachweisbar.

In Abb. 3 und 4: arachidonic acid = Arachidonsäure

## Patentansprüche

1. DNA-Sequenz, die für ein Protein mit der enzymatischen Aktivität einer Δ5-Desaturase kodiert,
**dadurch gekennzeichnet, dass** die enzymatische Aktivität ausschließlich für Dihomo-γ-Linolensäure spezifisch ist und die DNA-Sequenz ausgewählt ist aus der Gruppe bestehend aus
a) DNA-Sequenzen, die eine Nukleotidsequenz umfassen, die die in SEQ ID No. 2 angegebene Aminosäuresequenz oder Fragmente davon kodiert;
b) DNA-Sequenzen, die die in SEQ ID No. 1 angegebene kodierende Nukleotidsequenz oder Fragmente davon umfassen;
c) DNA-Sequenzen, die eine Nukleotidsequenz, die mit einem komplementären Strang der kodierenden Nukleotidsequenz von a) oder b) hybridisieren, oder Fragmente dieser Nukleotidsequenz umfassen;
d) DNA-Sequenzen, die eine Nukleotidsequenz, die zu einer Nukleotidsequenz von c) degeneriert ist, oder Fragmente dieser Nukleotidsequenz umfassen.

2. DNA-Sequenz nach Anspruch 1 aus *Phytophthora megasperma* stammend.

3. Rekombinantes Nukleinsäuremolekül, umfassend die folgenden Elemente:
- regulatorische Sequenzen eines in einer Zielzelle, vorzugsweise Pflanzen- oder Hefezelle, aktiven Promotors;
- operativ daran gebunden eine DNA-Sequenz nach einem der Ansprüche 1 oder 2;
- ggf. operativ daran gebunden regulatorische Sequenzen, die in der Zielzelle als Transkriptions-, Terminations- und/oder Polyadenylierungssignale dienen können.

4. Rekombinantes Protein mit der enzymatischen Aktivität einer Δ5-Desaturase, **dadurch gekennzeichnet, dass** die enzymatische Aktivität ausschließlich für Dihomo-γ-Linolensäure spezifisch ist und das Protein durch die DNA-Sequenz nach Anspruch 1 kodiert wird.

5. Rekombinantes Protein nach Anspruch 4 aus *Phytophthora megasperma* stammend.

6. Verfahren zur Erzeugung von Pflanzen bzw. Pflanzenzellen oder Hefezellen mit einem gegenüber Wildtypflanzen bzw. -zellen erhöhten Gehalt an Arachidonsäure, umfassend die folgenden Schritte:
a) Herstellung eines rekombinanten Nukleinsäuremoleküls, umfassend die folgenden Bestandteile in 5' -> 3'-Orientierung:
- regulatorische Sequenzen eines in Pflanzenzellen oder Hefezellen aktiven Promotors,
- operativ daran gebunden eine DNA-Sequenz nach einem der Ansprüche 1 oder 2 und
- ggf. operativ daran gebunden Sequenzen, die als Transkriptions-, Terminations- und/oder Polyadenylierungssignale in Pflanzenzellen oder Hefezellen dienen können.
b) Übertragung des Nukleinsäuremoleküls aus a) auf Pflanzenzellen oder Hefezellen; und
c) ggf. Regeneration von Pflanzen aus den transformierten Pflanzenzellen.

7. Transgene Pflanzenzellen oder transgene Hefezellen, enthaltend eine DNA-Sequenz oder ein rekombinantes Nukleinsäuremolekül nach einem der Ansprüche 1 bis 3, oder hergestellt nach einem Verfahren nach Anspruch 6, wobei die Zellen eine im Vergleich zu Wildtypzellen erhöhte Arachidonsäuresynthese aufweisen.

8. Transgene Pflanzen, enthaltend eine Pflanzenzelle nach Anspruch 7 oder hergestellt nach einem Verfahren nach Anspruch 6, sowie Teile dieser Pflanzen, transgene Ernteprodukte und transgenes Vermehrungsmaterial dieser Pflanzen, wie Protoplasten, Pflanzenzellen, Kalli, Samen, Knollen, Stecklinge, sowie die transgenen Nachkommen dieser Pflanzen.

9. Verfahren zur Herstellung von Arachidonsäure in transgenen Pflanzen bzw. Pflanzenzellen oder Hefezellen, umfassend die folgenden Schritte:
a) Übertragung einer DNA-Sequenz oder eines rekombinanten Nukleinsäuremoleküls nach einem der Ansprüche 1 bis 3 auf Pflanzen bzw. Pflanzenzellen oder Hefezellen,
b) Herstellung von Arachidonsäure durch Expression der DNA-Sequenz nach einem der Ansprüche 1 oder 2 in den transgenen Zellen, ggf. unter Zugabe von Dihomo-γ-Linolensäure zu den Zellen,
c) Gewinnung der Arachidonsäure aus den Zellen, Pflanzen oder Zellkulturen.

## Claims

1. A DNA sequence that codes for a protein having the enzymatic activity of a Δ5-desaturase,
**characterized in that** the enzymatic activity is specific solely for dihomo-γ-linolenic acid and the DNA sequence is selected from the group consisting of:
a) DNA sequences comprising a nucleotide sequence that codes for the amino acid sequence identified in SEQ ID No. 2 or fragments thereof;
b) DNA sequences comprising the coding nucleotide sequence given in SEQ ID No. 1 or fragments thereof;
c) DNA sequences comprising a nucleotide sequence, or fragments of said nucleotide sequence, that hybridize to a complementary strand of the coding nucleotide sequence from a) or b);
d) DNA sequences comprising a nucleotide sequence, or fragments of said nucleotide sequence, which is degenerated to a nucleotide sequence from c).

2. The DNA sequence according to claim 1 originating from *Phytophthora megasperma.*

3. A recombinant nucleic acid molecule comprising the following elements:
- regulatory sequences of a promoter which is active in a target cell, preferably a plant cell or a yeast cell;
- operatively linked thereto a DNA sequence according to any of claims 1 or 2;
- optionally, operatively linked thereto regulatory sequences, which can serve as transcription, termination and/or polyadenylation signals in the target cell.

4. A recombinant protein having the enzymatic activity of a Δ5-desaturase, **characterized in that** the enzymatic activity is specific solely for dihomo-γ-linolenic acid and the protein is coded by the DNA sequence according to claim 1.

5. The recombinant protein according to claim 4, originating from *Phytophthora megasperma.*

6. A method for generating plants or plant cells or yeast cells having an increased content of arachidonic acid compared to wild-type plants or wild-type cells, comprising the following steps:
a) producing a recombinant nucleic acid molecule comprising the following elements in 5' → 3' direction:
- regulatory sequences of a promoter which is active in plant cells or yeast cells,
- operatively linked thereto a DNA sequence according to any of claims 1 or 2, and
- optionally, operatively linked thereto sequences, which can serve as transcription, termination and/or polyadenylation signals in plant cells or yeast cells,
b) transferring the nucleic acid molecule from a) to plant cells or yeast cells; and
c) optionally, regenerating plants from the transformed plant cells.

7. Transgenic plant cells or transgenic yeast cells containing a DNA sequence or a recombinant nucleic acid molecule according to any of claims 1 to 3 or produced by a method according to claim 6, whereby the cells have an increased arachidonic acid synthesis compared to wild-type plants.

8. Transgenic plants containing a plant cell according to claim 7 or produced by a method according to claim 6 as well as parts of these plants, transgenic harvest products and transgenic propagating material of these plants, such as protoplasts, plant cells, calli, seeds, tubers, cuttings, and the transgenic progeny of these plants.

9. A method for producing arachidonic acid in transgenic plants or plant cells or yeast cells, comprising the following steps:
a) transferring a DNA sequence or a recombinant nucleic acid molecule according to any of claims 1 to 3 to plants or plant cells or yeast cells,
b) producing arachidonic acid by expression of the DNA sequence according to any of claims 1 or 2 in the transgenic cells, optionally by addition of dihomo-γ-linolenic acid to these cells,
c) obtaining arachidonic acid from these cells, plants or cell cultures.

## Revendications

1. Séquence ADN qui code pour une protéine avec l'activité enzymatique d'une Δ5 désaturase,
**caractérisée en ce que** l'activité enzymatique est spécifique exclusivement pour de l'acide dihomo γ linolénique et que la séquence ADN est sélectionnée dans le groupe se constituant de :
a) séquences ADN qui comprennent une séquence de nucléotides qui code la séquence d'acides aminés indiquée dans SEQ ID n° 2 ou des fragments de celle-ci ;
b) séquences ADN qui comprennent la séquence de nucléotides codante indiquée dans SEQ ID n° 1 ou des fragments de celle-ci ;
c) séquences ADN qui comprennent une séquence de nucléotides qui hybride avec un brin complémentaire de la séquence de nucléotides codante de a) ou de b) ou des fragments de cette séquence de nucléotides ;
d) séquences ADN qui comprennent une séquence de nucléotides qui a dégénéré en une séquence de nucléotides de c) ou des fragments de cette séquence de nucléotides.

2. Séquence ADN selon la revendication 1, provenant de *phytophthora megasperma.*

3. Molécule d'acide nucléique recombinant comprenant les éléments suivants :
- des séquences de régulation d'un promoteur actif dans une cellule cible, de préférence une cellule de plante ou de levure ;
- une séquence ADN liée opérationnellement, selon l'une quelconque des revendications 1 ou 2 ;
- des séquences de régulation éventuellement liées opérationnellement qui peuvent servir en tant que signaux de transcription, de terminaison et/ou de polyadénylation dans la cellule cible.

4. Protéine recombinante avec l'activité enzymatique d'une Δ5 désaturase **caractérisée en ce que** l'activité enzymatique est spécifique exclusivement pour de l'acide dihomo γ linolénique et que la protéine est codée par la séquence ADN selon la revendication 1.

5. Protéine recombinante selon la revendication 4, provenant de *phytophthora megasperma.*

6. Procédé de génération de plantes ou de cellules de plante ou de cellules de levure avec une teneur plus importante d'acide arachidonique par rapport à des plantes sauvages ou des cellules de plantes sauvages, qui comprend les étapes suivantes :
a) fabrication d'une molécule d'acide nucléique recombinant comprenant les composants suivants en orientation 5' -> 3' :
- des séquences de régulation d'un promoteur actif dans des cellules de plante ou des cellules de levure,
- une séquence ADN liée opérationnellement selon l'une quelconque des revendications 1 ou 2 et
- éventuellement des séquences liées opérationnellement qui peuvent servir en tant que signaux de transcription, de terminaison et/ou de polyadénylation dans des cellules de plante ou des cellules de levure,
b) transfert de la molécule d'acide nucléique de a) vers des cellules de plante ou des cellules de levure ; et
c) éventuellement régénération de plantes à partir des cellules de plante transformées.

7. Cellules de plante transgénique ou cellules de levure transgénique contenant une séquence ADN ou une molécule d'acide nucléique recombinant selon l'une quelconque des revendications 1 à 3 ou fabriquées selon un procédé selon la revendication 6, les cellules présentant une synthèse supérieure d'acide arachidonique par rapport à des cellules sauvages.

8. Plantes transgéniques contenant une cellule de plante selon la revendication 7 ou fabriquées selon un procédé selon la revendication 6, ainsi que des parties de ces plantes, des produits agricoles transgéniques et des matériaux de reproduction transgénique de ces plantes tels des protoplasmes, des cellules de plantes, des cals, des semences, des bulbes, des plants ainsi que des descendants transgéniques de ces plantes.

9. Procédé de fabrication d'acide arachidonique dans des plantes ou des cellules de plante ou des cellules de levure transgéniques comprenant les étapes suivantes :
a) transfert d'une séquence ADN ou d'une molécule d'acide nucléique recombinant selon l'une quelconque des revendications 1 à 3 vers des plantes ou des cellules de plante ou des cellules de levure,
b) fabrication d'un acide arachidonique par expression de la séquence ADN selon l'une quelconque des revendications 1 ou 2 dans les cellules transgéniques éventuellement avec apport d'acide dihomo γ linolénique aux cellules,
c) prélèvement de l'acide arachidonique des cellules, plantes, ou cultures de cellules.
